# EUROPEAN PATENT APPLICATION

(11) **EP 1 604 696 A1**
(43) Date of publication of application: **14.12.2005**
(21) Application number: 05076538.7
(22) Date of filing: 26.06.2002
(51) Int. Cl.: A61L 27/44, A61L 27/56, A61L 27/42, A61L 27/40

(54) **Porous ceramic/porous polymer layered scaffolds for the repair and regeneration of tissue**

(30) Priority: 27.06.2001 US 892993
(62) Divisional of application: 02254457.1
(71) Applicant: ETHICON, INC., Somerville, NJ 08876 (US)
(72) Inventor: Brown, Kelly R., Hillsborough NJ 08844 (US); Yuan, Jenny J., Neshanic Station NJ 08853 (US); Li, Yufu, Bridgewater NJ 08807 (US); Zimmerman, Mark C., East Brunswick NJ 08816 (US)
(74) Representative: Mercer, Christopher Paul

(57) **Abstract**

A composite scaffold with a porous ceramic phase and a porous polymer phase. The polymer is foamed while in solution that is infused in the pores of the ceramic to create a interphase junction of interlocked porous materials. The preferred method for foaming is by lyophilization. The scaffold may be infused or coated with a variety of bioactive materials to induce ingrowth or to release a medicament. The multi-layered porous scaffold can mimic the morphology of an injured tissue junction with a gradient morphology and cell composition, such as articular cartilage.

## Description

### Field of the Invention

The present invention relates generally to the field of tissue repair, and more particularly to porous biocompatible ceramics, polymers and composites for use in tissue scaffolds.

### Background of the Invention

Porous ceramic materials such as hydroxyapatite, soluble glasses and ceramic foams have been used as scaffolds for the ingrowth of tissue due to compositional and morphological biocompatability. For example, the porosity of such materials promotes cell infiltration. A variety of methods are used to prepare porous ceramic scaffolds (prostheses), such as hydrothermally treating animal bone or coral, burning off polymer beads mixed into a ceramic body, vapor deposition on foam, infiltration of polymer foam with a ceramic slip and foaming a ceramic slip.

One limitation exhibited by porous ceramic materials is their inherent brittleness. Attempts to address this limitation have included back-filling a ceramic foam with monomer solutions of PMMA or PLA, draining excess solution from the ceramic foam then polymerizing through curing and/or drying in order to impart some toughness to the ceramic foam. Others have proposed laminating solid or porous polymeric layers to a ceramic foam structure.

Independent from proposed uses in combination with ceramics, polymeric foams have utility in the repair and regeneration of tissue. For example, amorphous, polymeric foam has been used to fill voids in bone. Various methods have been explored for preparing the polymer foams, using, e.g., leachables; vacuum foaming techniques; precipitated polymer gel masses; and polymer melts with fugitive compounds that sublime at temperatures greater than room temperature. The formation of biocompatible absorbable foams by lyophilization is discussed in EP-A-1 064 958.

Hinsch et al. (EP0274898) describes a porous open cell foam of polyhydroxy acids for the in-growth of blood vessels and cells. The foam can be reinforced with fibers, yarns, braids, knitted fabrics, scrims and the like.

Athanasiou et al. (U.S. Patent No. 5,607,474) have proposed using a two-layer polymeric foam device for repairing osteochondral defects at a location where two dissimilar types of tissue are present. The two polymeric layers are prepared separately, and joined together at a subsequent step. Each of the layers is designed to have stiffness and compressibility values that correspond respectively to cartilage and bone tissue, mimicking the cartilage/bone interface. However, the Athanasiou device exhibits an abrupt change in properties from one layer to the next, whereas the juncture of cartilage and bone displays a gradual transition, with cartilage cells gradually changing cell morphology and orientation depending on the location relative to the underlying bone structure. Further, collagen fiber orientation within the matrix also changes relative to its location in the structure.

H. Levene et al., U.S. Patent No. 6,103,255 describes a process used for making a scaffold having a substantially continuous polymer phase with a distribution of large and small pore sizes, with the small pores contained in the walls of the large pores.

In a study done by G. Niederauer et al. and reported in *Biomaterials* 21 (2000) 2561, scaffolds for articular cartilage repair were prepared from layers of polylactic/polyglycolic acid (PLG) and polylactic/polyglycolic acid reinforced with fibers of the same material, bioglass or calcium sulfate. The PLG layer was made porous in all cases by expanding a precipitated gel mass of polymer under vacuum at elevated temperatures. The reinforced layers were made porous in a similar fashion after incorporating the reinforcement in the polymer solution and prior to precipitation of the polymeric gel mass. Once the two layers were fabricated, they were adjoined using a small amount of solvent to glue the two layers together.

The use of a porous polymer for the purpose of engineering cartilage is described in the patent by T. Mahood et al. (EP1027897A1) which discloses a multi-layer polymer scaffold in which the layers are attached by successive dip coating or by the attachment of the two layers to a third. The third layer is described as a barrier to cell diffusion, thus confining chondrocytes to the polymer layer and osteoblasts to the ceramic layer.

Kreklau et al. in *Biomaterials* 20 (1999) 1743 have evaluated a fibrous polymeric fleece attached to a porous ceramic material, for the purpose of culturing chondrocytes in the polymeric scaffold while simultaneously providing a bone formation inducing absorbable material to simulate articular cartilage. In this study, a fibrin-cell-solution was used to affix the ceramic and polymeric layers by way of encapsulation with the intent that the phases would interact *in vitro* in order to create a mechanically stressable junction. The authors discuss the possibility of providing the surfaces of the layers with teeth to increase shear strength. However, there is no mechanism by which the two different layers are interlocked to resist delaminating forces in directions perpendicular to the laminate function and there is an abrupt transition between the two layers.

It would be advantageous to overcome the above mentioned limitations of known scaffolds.

### Summary of Invention

The limitations of the prior art are overcome by the present invention which includes a composite scaffold with a ceramic phase having a first plurality of pores and a polymer phase having a second plurality of pores. The polymer phase is attached to the ceramic phase at an interphase region with the polymer phase infused at least partially into the first plurality of pores in the interphase region. In accordance with a method for making the scaffold, the ceramic phase is placed in contact with a polymer solution which infuses into the pores of the ceramic phase. The polymer solution is then foamed to produce a polymer foam interlocked with the ceramic phase.

### Brief Description of Figures

Figure 1 is a perspective view of a composite scaffold in accordance with a first embodiment of the present invention;
Figure 2 is a cross-sectional view of the scaffold of Figure 1 taken along section line II-II and looking in the direction of the arrows;
Figure 3 is a perspective view of a scaffold in accordance with a second exemplary embodiment of the present invention;
Figure 4 is a cross-sectional view of the scaffold of Figure 3 taken along section line IV-IV and looking in the direction of the arrows;
Figure 5 is a perspective, partially phantom view of a scaffold in accordance with a third exemplary embodiment of the present invention;
Figure 6 is a perspective view of a truss structure incorporated in the scaffold of Figure 5;
Figure 7 is an enlarged view of a segment VII of the truss structure of Figure 6;
Figure 8 is a perspective view of an alternative truss structure that may be incorporated in a scaffold in accordance with the present invention; and
Figure 9 is a scanning electron micrograph of the cross-section of the porous ceramic/porous polymer interphase region of a scaffold in accordance with the present invention, such as shown in Figure 1.

### Detailed Description of the Invention

The present invention relates to bi- or multi-layered scaffolds with a porous, bioabsorbable polymer layer attached to a porous ceramic layer via a porous transitional interface. The scaffolds are particularly useful in the repair/regeneration of defects present at a junction of tissue types exhibiting a transitional or gradient morphology/physiology such as at the cartilage/bone junction. The present invention can be utilized to repair/regenerate a tissue junction by inducing one cell type to proliferate in the polymer phase of the scaffold and a second cell type to grow in the ceramic phase of the scaffold. Examples of such junction regeneration sites are (a) spinal disc (nuclear and annular cells cultured on the polymer phase and osteoblasts cultured in the ceramic phase); (b) articular or meniscal cartilage (chondrocytes, or fibrochondrocytes, respectively, cultured on the polymer phase and osteoblasts cultured in the ceramic). The present invention may also be utilized to repair the meniscus, fibrocartilage, tendons, and ligaments. The features of the porous polymer phase can be controlled to suit a desired application by choosing the appropriate conditions during the process of lyophilization, or freeze drying. The porous polymer foam can be directly lyophilized into the ceramic structure creating a multiphasic material composed of a polymer foam with or without reinforcement structures, an interphase zone of polymer foam diffused and expanded within the porous ceramic, and a porous ceramic. These features in partially or completely absorbable implants have advantages over the prior art where the scaffolds are typically isotropic or random structures.

One of the major weaknesses of the prior art regarding laminated scaffolds is that the layers are not completely integrated and are subject to delamination under *in vivo* conditions. The present invention solves the problem of delamination by the lyophilization of a porous polymer foam in the presence of a porous ceramic, interlocking the porous polymeric foam with the porous ceramic by way of an interphase zone of porous polymer infiltrated into the porous ceramic. This interphase zone exhibits a microporous polymer foam located within the macropores of a porous ceramic. The interpenetration of the two porous layers creates a strong mechanical junction while simultaneously providing a gradual change in material properties for the purpose of regenerating different tissues or co-culturing different types of cells in intimate contact with one another. The interconnecting pores and channels facilitate the transport of nutrients and/or invasion of cells into the scaffold, facilitating the ingrowth of tissue. This transitional composite structure more closely mimics naturally occurring tissue junctions. The present invention therefore facilitates cellular organization and the regeneration of tissue junctions with normal morphology and physiology.

The features of a scaffold in accordance with the present invention can be tailored to suit a particular application by selecting the appropriate ceramic, polymer and conditions for lyophilization of the polymer to obtain one or more of the following properties: (1) interconnecting polymer foams attached to the porous ceramic (2) a variety of porosities ranging from about 20% to about 98% for the polymer foam; (3) a gradient in the pore size between the polymer and ceramic; (4) channels that run through the porous polymer foam for improved cell invasion, vascularization and nutrient diffusion; and (5) micro-patterning of pores or the addition of other polymer structures on the surface of the polymer for cellular organization or to limit cellular invasion.

In addition, the scaffold can include (1) porous composites with a composition gradient to elicit or take advantage of different cell response to different materials; (2) reinforcement with knitted, braided, woven, or non-woven fabrics or meshes, or truss structures in order to impart desired mechanical properties; (3) blends of different polymer compositions to create a polymer phase that has portions that will break down at different rates; (4) multi-layer composite structures with layers of alternating porous ceramics and polymers; (5) a polymer phase co-lyophilized or coated with pharmaceutically active compounds; (6) a ceramic phase coated with pharmaceutically active compounds such as growth factors; and/or (7) cells which may be cultured prior to or at the time of implantation.

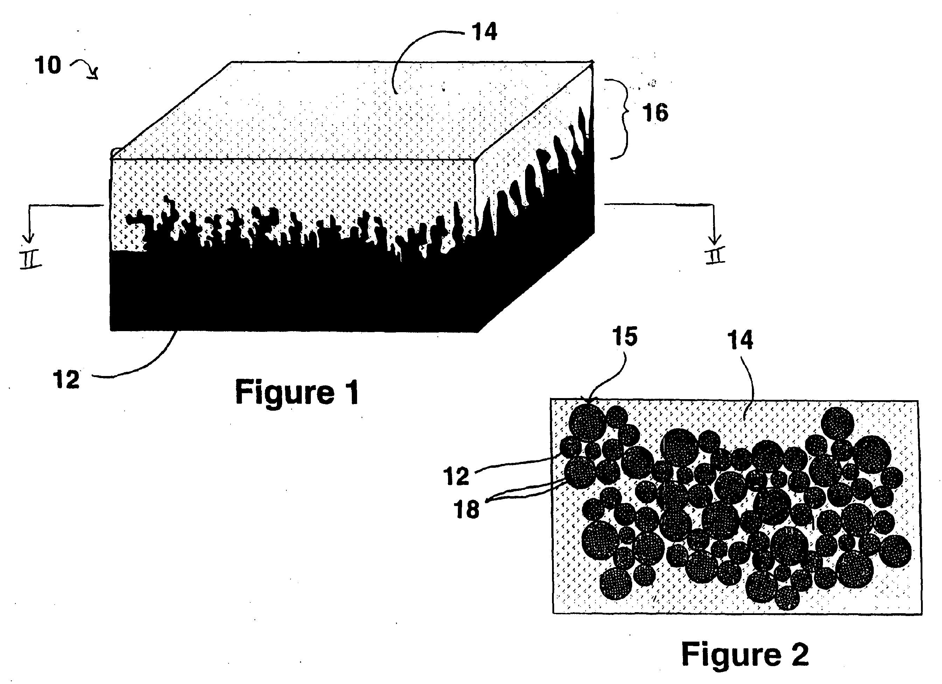

Figures 1 and 2 show a composite scaffold 10 with a porous polymer phase 12 and porous ceramic phase 14, which are mechanically interlocked at interphase region 16. In the interphase region 16 shown in the sectional view of Figure 2, macropores 15 in ceramic phase 14 are filled with polymer phase 12. Polymer phase 12, in turn contains micropores 18.

The infusion of the polymer phase 12 into the ceramic phase 14 securely fastens the two phases 12, 14 and supports the brittle structure of the porous ceramic phase 14. The polymer phase 12 acts as a cushion to dissipate impact energy to shield the brittle ceramic phase 14 from catastrophically damaging stresses. In addition, the communicating pores 15, 18 encourage the growth of different types of cells, promoting the regeneration of different adjoining layers of tissue at an injured tissue junction.

The macropores 15 in the ceramic phase 14 are interconnected, and may be selected to have pore sizes ranging from 25 to 600 microns, preferably from 100 to 250 microns. The micropores 18 in the polymer phase 12 are also interconnected and range in size from about 10 to 250 microns, preferably 30 to 150 microns. The terms "micropore" and "macropore" are used to designate the two size scales of pores found in the scaffold 10. If the brittle ceramic phase 14 is cracked, the polymer phase 12 in the interphase region 16 holds the scaffold together. The composite scaffold 10 facilitates the creation of a strong bond between different types of tissue such as in the repair and regeneration of articular cartilage, meniscus, and spinal discs.

The scaffold 10 may be made by infiltrating the porous ceramic phase 14 with the desired elastomeric polymer phase 12, and then causing the polymer phase 12 to foam. The polymer phase 12 may be foamed by lyophilization, supercritical solvent foaming (i.e., as described in EP 464163B1), gas injection extrusion, gas injection molding or casting with an extractable material, e.g., salts, sugar or by any other means known to those skilled in the art. Currently, it is preferred to foam the polymer 12 by lyophilization (freeze drying). Suitable methods for lyophilizing elastomeric polymers to form foams are described in EP-A-1 064 958.

The first step in making the scaffold 10 is to dissolve the desired elastomeric polymer to form phase 12 in a solvent, forming a polymer solution. The polymer solution is brought into contact with the porous ceramic phase 14 and the low viscosity polymeric solution wicks via capillary action into the pores of the ceramic layer 14. Other infiltrating methods include injecting the solution into the ceramic layer 14 under pressure and vacuum assisted infiltration.

In the wicking method, the ceramic phase 14 is only partially submerged in polymer solution, which is contained in a receptacle or mold. The mold may be made from any material that does not interfere with the polymer-solvent system and is preferably formed from a heat conductive material. The preferred method of partially submerging the ceramic phase 14 is to place shims (spacers) on the inside bottom of the mold, and place the ceramic layer on the shims. The thickness of the spacers and the volume of polymer charged in the mold controls the thickness of the polymer phase 12 and the interphase region 16.

The above-described mold assembly is then placed in a freeze dryer and subject to directional cooling (through the wall of the mold that is in contact with the freeze dryer shelf) in a thermal cycle. The heat transfer front moves upwards from the lyophilizer shelf through the mold and into the polymer solution. When the temperature of the biopolymer solution goes below the gelation and/or freezing point, it separates into polymer and solvent phases giving rise to the cell/foam structure.

The pore morphology that results from the freezing step is a function of solution thermodynamics, freezing rate, temperature to which it is cooled, concentration of the solution, the presence of reinforcement elements, the presence of an adjoining layer, the occurrence of homogeneous or heterogeneous nucleation, etc. Detailed descriptions of these phase separation phenomena are known in the art and can be found in the references "Microcellular foams via phase separation" by A. T. Young, *J. Vac. Sci. Technol*., A 4(3), May/Jun 1986; and "Thermodynamics of Formation of Porous Polymeric Membrane from Solutions" by S. Matsuda, *Polymer J.* 23(5), (1991) 435. The lyophilization process can therefore be used to bond the polymer and ceramic layers 12, 14 while simultaneously creating a composite material with the correct pore structure to regenerate a tissue function.

The porous ceramic phase 14 of the scaffold may be composed of mono-, di-, tri-, ∀-tri, 3-tri, and tetra-calcium phosphate, hydroxyapatite, fluoroapatites, calcium sulfates, calcium fluorides, calcium oxides, calcium carbonates, magnesium calcium phosphates, bioglasses, and mixtures thereof. There are a number of suitable porous biocompatible ceramic materials currently available on the commercial market such as Surgibone (Unilab Surgibone, Inc., Canada), Endobon (Merck Biomaterial France, France), Ceros (Mathys, A. G., Bettlach, Switzerland), and Interpore (Interpore, Irvine, CA, United States).

Alternatively, the ceramic phase 14 may be in the form of a porous polymer matrix with inclusions of short ceramic fibers or particulates. This alternative ceramic phase 14 may be formed by conventional methods for working plastics, such as injection molding, with the porosity thereof provided by leachable inclusions, molds with pore forming pins, or drilling.

The polymeric layer 12 may be either a natural biopolymer, a synthetic polymer, or combinations of both. Natural biopolymers include collagen, elastin, alginate, chitin, hyaluronic acid, and others. Examples of suitable synthetic biocompatible, bioabsorbable polymers that could be used include aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, biomolecules and blends thereof.

For the purpose of this invention aliphatic polyesters include but are not limited to homopolymers and copolymers of lactide (which includes lactic acid, D-,L- and meso lactide), glycolide (including glycolic acid), ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, 1,4-dioxepan-2-one (including its dimer 1,5,8,12-tetraoxacyclotetradecane-7,14-dione), 1,5-dioxepan-2-one, 6,6-dimethyl-1,4-dioxan-2-one, 2,5-diketomorpholine, pivalolactone, alpha, alpha-diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one and polymer blends thereof.

Poly(iminocarbonates) for the purpose of this invention include those described by Kemnitzer and Kohn, in the Handbook of Biodegradable Polymers, edited by Domb, Kost and Wisemen, Hardwood Academic Press, 1997, pages 251-272. Copoly(ether-esters) for the purpose of this invention include those copolyester-ethers described by Cohn and Younes *J*. *Biomater. Res.,* 22, (1988) 993, and Cohn, *Polymer Preprints,* 30(1), (1989) 498.

Polyalkylene oxalates for the purpose of this invention include those described in U.S. Patent Nos. 4,208,511; 4,141,087; 4,130,639; 4,140,678; 4,105,034; and 4,205,399.

Polyphosphazenes for the purpose of this invention include co-, ter- and higher order mixed monomer based polymers made from L-lactide, D,L-lactide, lactic acid, glycolide, glycolic acid, para-dioxanone, trimethylene carbonate and ε-caprolactone those described by Allcock in The Encyclopedia of Polymer Science, Wiley Intersciences, John Wiley & Sons, 13 (1988) 31, and by Vandorpe, Schacht, Dejardin and Lemmouchi in the Handbook of Biodegradable Polymers, edited by Domb, Kost and Wisemen, Hardwood Academic Press, (1997) 161.

Polyanhydrides for the purpose of this invention include those from diacids of the form HOOC-C₆H₄-O-(CH₂)ₘ-O-C₆H₄-COOH where m is an integer in the range of from 2 to 8 and copolymers thereof with aliphatic alpha-omega diacids of up to 12 carbons.

Polyoxaesters, polyoxaamides and polyoxaesters containing amines and/or amino groups for the purpose of this invention include those described in one or more of the following U.S. Patent Nos. 5,464,929; 5,595,751; 5,597,579; 5,607,687; 5,618,552; 5,620,698; 5,645,850; 5,648,088; 5,698,213; 5,700,583; and 5,859,150. Polyorthoesters for the purpose of this invention include those described by Heller in the Handbook of Biodegradable Polymers, edited by Domb, Kost and Wisemen, Hardwood Academic Press, (1997),99.

Aliphatic polyesters are preferred for making the polymers phase 12 of the scaffold 10. Aliphatic polyesters can be homopolymers, copolymers (random, block, segmented, tapered blocks, graft, triblock, etc.) having a linear, branched or star structure. The preferred morphology of the copolymer chains is linear. Suitable monomers for making aliphatic homopolymers and copolymers may be selected from the group consisting of, but not limited to, lactic acid, lactide (including L-, D-, meso and D,L mixtures), glycolic acid, glycolide, ε-caprolactone, p-dioxanone (1,4-dioxan-2-one), trimethylene carbonate (1,3-dioxan-2-one), delta-valerolactone, beta-butyrolactone, epsilon-decalactone, 2,5-diketomorpholine, pivalolactone, alpha, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 6,6-dimethyl-dioxepan-2-one, 6,8-dioxabicycloctane-7-one and combinations thereof.

Elastomeric copolymers also are particularly useful in the present invention. Suitable bioabsorbable, biocompatible elastomers include, but are not limited to, those selected from the group consisting of elastomeric copolymers of ε-caprolactone and glycolide (preferably having a mole ratio of ε-caprolactone to glycolide of from about 35:65 to about 65:35, more preferably from 45:55 to 35:65); elastomeric copolymers of ε-caprolactone and lactide, including L-lactide, D-lactide blends thereof or lactic acid copolymers (preferably having a mole ratio of ε-caprolactone to lactide of from about 35:65 to about 65:35 and more preferably from 45:55 to 30:70 or from about 95:5 to about 85:15); elastomeric copolymers of p-dioxanone (1,4-dioxan-2-one) and lactide including L-lactide, D-lactide and lactic acid (preferably having a mole ratio of p-dioxanone to lactide of from about 40:60 to about 60:40); elastomeric copolymers of ε-caprolactone and p-dioxanone (preferably having a mole ratio of ε-caprolactone to p-dioxanone of from about from 30:70 to about 70:30); elastomeric copolymers of p-dioxanone and trimethylene carbonate (preferably having a mole ratio of p-dioxanone to trimethylene carbonate of from about 30:70 to about 70:30); elastomeric copolymers of trimethylene carbonate and glycolide (preferably having a mole ratio of trimethylene carbonate to glycolide of from about 30:70 to about 70:30); elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30); and blends thereof. Examples of suitable bioabsorbable elastomers are also described in U.S. Patent Nos. 4,045,418, 4,057,537 and 5,468,253.

In the preferred embodiments of this invention, the elastomer from which the foams are formed will exhibit a percent elongation greater than about 200 percent and preferably greater than about 500 percent. The properties that determine the degree of elasticity of the bioabsorbable elastomer are achieved while maintaining a tensile strength greater than about 500 psi, preferably greater than about 1,000 psi, and a tear strength of greater than about 50 lbs/inch, preferably greater than about 80 lbs/inch.

The polymers or copolymer suitable for forming the polymers phase 12 of the implant 10 for any particular application depends on several factors. The chemical composition, spatial distribution of the constituents, the molecular weight of the polymers and the degree of crystallinity, all dictate to some extent the *in vitro* and *in vivo* behavior of the polymer. However, the selection of the polymer to make gradient foams for tissue regeneration largely depends on (but is not limited to) the following factors: (a) bioabsorption (or biodegradation) kinetics; (b) *in vivo* mechanical performance; (c) cell response to the material in terms of cell attachment, proliferation, migration and differentiation and (d) biocompatibility.

The ability of the polymer phase to resorb in a timely fashion *in vivo* is critical. The differences in the absorption time under *in vivo* conditions can also be the basis for combining two different copolymers. For example, a copolymer of 35:65 ε-caprolactone and glycolide (a relatively fast absorbing polymer) is blended with 40:60 ε-caprolactone and (L)lactide copolymer (a relatively slow absorbing polymer) to form a foam. Such a foam could be processed to yield several different physical structures depending upon the technique used. The two constituents can be either randomly inter-connected bicontinuous phases, or have a gradient or laminate composition with an integrated interface between the constituent layers. The microstructure of these foams can be optimized to regenerate or repair the desired anatomical features of the tissue that is being engineered.

Suitable solvents for the preferred absorbable aliphatic polyesters that will not affect the ceramic foams include but are not limited to solvents selected from a group consisting of formic acid, ethyl formate, acetic acid, hexafluoroisopropanol (HFIP),cyclic ethers (i.e. THF, DMF, and PDO), acetone, acetates of C2 to C5 alcohol (such as ethyl acetate and t-butylacetate), glyme (i.e. monoglyme, ethyl glyme, diglyme, ethyl diglyme, triglyme, butyl diglyme and tetraglyme) methylethyl ketone, dipropyleneglycol methyl ether, lactones (such as γ-valerolactone, δ-valerolactone, β-butyrolactone, γ-butyrolactone) 1,4-dioxane, 1,3-dioxolane, 1,3-dioxolane-2-one (ethylene carbonate), dimethlycarbonate, diethylcarbonate, benzene, toluene, benzyl alcohol, p-xylene, naphthalene, tetrahydrofuran, N-methyl pyrrolidone, dimethylformamide, chloroform, 1,2-dichloromethane, morpholine, dimethylsulfoxide, hexafluoroacetone sesquihydrate (HFAS), anisole and mixtures thereof. Among these solvents, the preferred solvent is 1,4-dioxane. A homogeneous solution of the polymer in the solvent is prepared using standard techniques.

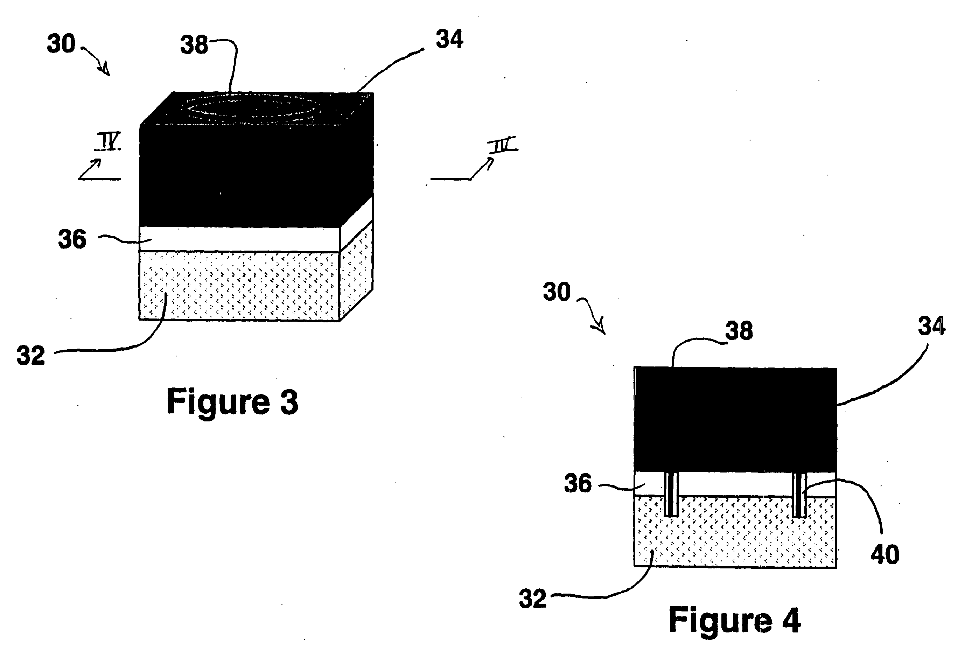

Additionally, the polymer 12 can include reinforcements such as films, scrims, woven, nonwoven, knitted or braided textile structures. Figures 3 and 4 show a composite scaffold 30 with polymer phase 34 and ceramic phase 32 mechanically interlocked at interphase region 36 (shown diagrammatically as a discreet layer, but having the interspersed configuration of interface layer 16 of FIG. 1). Tubular reinforcement 38, is first inserted into ceramic phase 32 by means of groove 40 machined into the ceramic phase, and subsequently embedded in polymer phase 34 during the lyophilization process.

In addition to altering the mechanical properties of the scaffold 30, reinforcement 38 or truss structure 70 (described below) can be utilized: (i) to modify the *in-vitro* behavior of the scaffold 30, e.g., by introducing a different *in-vitro* profile; (ii) as a carrier for the controlled release of a drug; and (iii) as a carrier for Micro-Electro Mechanical Systems (MEMS).

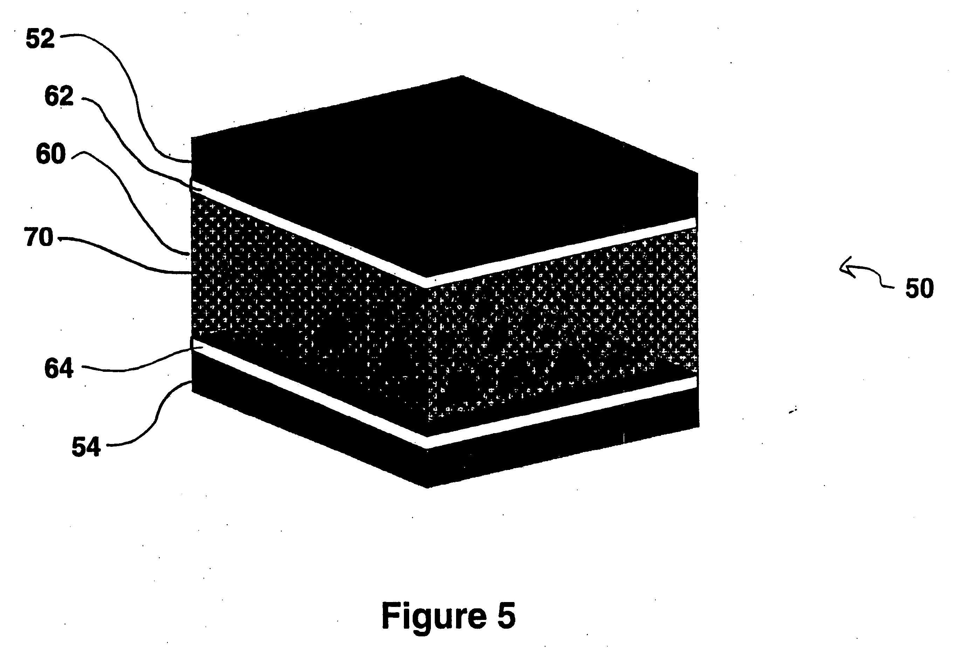

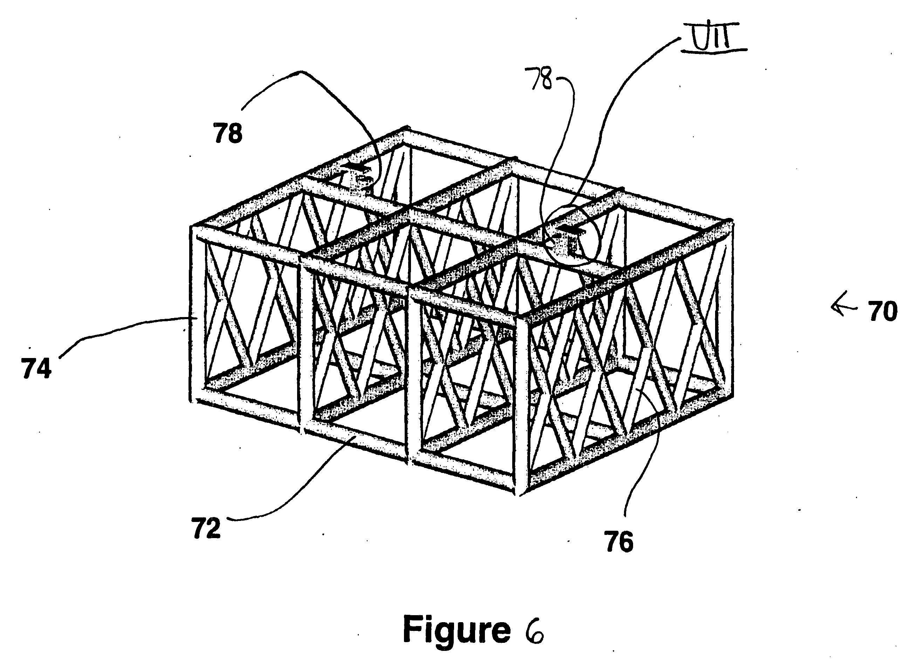

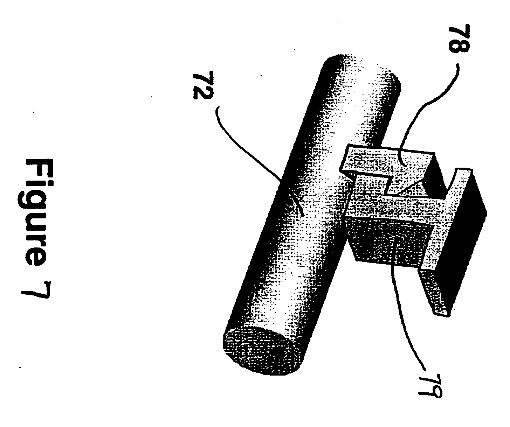

Similarly, truss structures may be incorporated into the scaffold 10, 30. Figure 5 shows composite scaffold 50 having ceramic phases 52 and 54, polymer phase 60, and truss structure 70 (in phantom). Ceramic phases 52 and 54 are mechanically interlocked with polymer phase 60 at interphase regions 62 and 64 as in the embodiments described above. Figure 6 and 7 show truss structure 70, having horizontal supports 72, vertical supports 74, angular supports 76, and locking tabs 78. Truss structure 70 may be fabricated from a polymer (preferably an absorbable polymer), ceramic or composite, as listed above, by injection molding or other methods, such as stereolithography or 3-D printing, to achieve complex three-dimensional structures. The truss structure 70 provides enhanced mechanical properties for scaffold 50, i.e., in resisting compression, bending and shearing. Locking device 78 is countersunk into ceramic phases 52 and 54 to strengthen the attachment of truss structure 70 thereto. Alternatively, locking device 78 may cooperate with a mating latch element 79 affixed or formed in ceramic phases 52, 54.

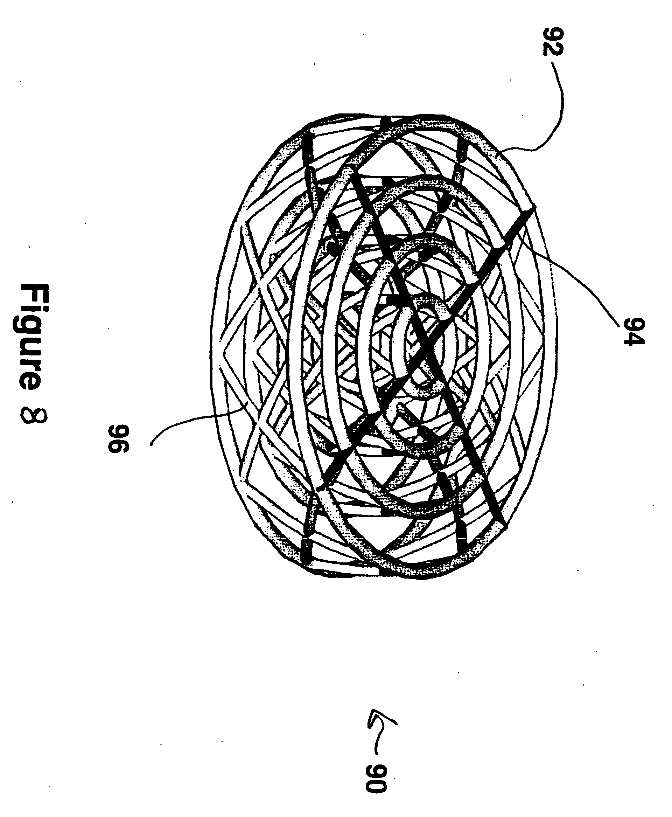

Figure 8 shows an alternative truss structure 90 that can be used in a generally cylindrically shaped scaffold (not shown) otherwise having the same configuration as scaffold 50 of Figure 5. Truss structure 90 has circular supports 92, linear supports 94, and angular supports 96.

Scaffolds 10, 30, 50 may be utilized in combination with a scaffold fixation device having a post insertable into a hole bored in bone and a scaffold support disposed at 90° relative to the post. The scaffolds 10, 30, 50 can be attached to a post-type fixation device by joining the scaffold support to the ceramic phase, e.g., 32 prior to lyophilization. Alternatively, the scaffold 10, 30, 50 may be affixed in the defect region by way of calcium phosphate or calcium sulfate cement, PMMA, fibrin glue, adhesives (such as cyanoacrylates or butyl acrylates) or simply by press fitting.

Solids may be added to the polymer layer e.g., 12, to promote tissue regeneration/regrowth, to act as buffers, reinforcing materials, porosity modifiers, and/or radio-opaque markers to allow imaging after implantation. Suitable solids include, but are not limited to, particles of demineralized bone, calcium phosphate particles, calcium carbonate particles for bone repair, leachable solids for pore creation and particles of bioabsorbable polymers not soluble in the solvent system as reinforcing agents or for the creation of pores as they are absorbed.

Suitable leachable solids include but are not limited to nontoxic leachable materials such as salts (i.e., sodium chloride, potassium chloride, calcium chloride, sodium tartrate, sodium citrate, and the like) biocompatible mono and disaccharides (i.e., glucose, fructose, dextrose, maltose, lactose and sucrose), polysaccharides (i.e., starch, alginate), water soluble proteins (i.e., gelatin and agarose) and paraffin. Generally all of these materials will have an average diameter of less than about 1 mm and preferably will have an average diameter of from about 50 to about 500 µm. The particles will generally constitute from about 1 to about 50 volume percent of the total volume of the particle and polymer-solvent mixture (wherein the total volume percent equals 100 volume percent). The leachable materials can be removed by immersing the foam with the leachable material in a solvent in which the particle is soluble for a sufficient amount of time to allow leaching of substantially all of the particles, but which does not dissolve or detrimentally alter the foam. The preferred extraction solvent is water, most preferably distilled-deionized water. This process is described in U.S. Patent No. 5,514,378. Preferably the foam will be dried after the leaching process is complete at low temperature and/or vacuum dried to minimize hydrolysis of the foam unless accelerated absorption of the foam is desired.

Various proteins (including short chain peptides), growth agents, chemotatic agents and therapeutic agents (antibiotics, analgesics, anti-inflammatories, anti-rejection (e.g. immunosuppressants) and anticancer drugs), or ceramic particles can be added to the composite scaffold 10, 30, 50 during processing or adsorbed onto the surface or back-filled into the scaffold 10 after fabrication. The pores of the ceramic phase 14, 32, 52, 54 and/or the polymer 12, 34, 60 may be partially or completely filled with biocompatible resorbable synthetic polymers or polymess (such as collagen or elastin) or biocompatible ceramic materials (such as hydroxyapatite) and combinations thereof (that may or may not contain materials that promote tissue growth). Suitable materials include but are not limited to autograft, allograft, or xenograft bone, bone marrow, morphogenic proteins (BMPs), epidermal growth factor (EGF), fibroblast growth factor (FGF), platelet derived growth factor (PDGF), insulin derived growth factor (IGF-I and IGF-II), transforming growth factors (TGF-β), vascular endothelial growth factor (VEGF), platelet rich plasma (PRP) or other osteoinductive or osteoconductive materials known in the art. The polymes fillers could also be conductive or chemotactic materials, or delivery vehicles for growth factors. Examples would be recombinant or animal derived collagen, elastin or hyaluronic acid.

Bioactive coatings or surface treatments could also be applied to the surface of the scaffold 10, 30, 50. For example, bioactive peptide sequences (RGDs) could be applied to facilitate protein adsorption and subsequent cell tissue attachment.

Therapeutic agents may also be delivered via the scaffold 10, 30, 50. The polymess and blends that are used to form the scaffold 10, 30, 50 can contain therapeutic agents. For example, polymes 12, 34, 60 would be mixed with a therapeutic agent prior to forming the composite scaffold 10, 30, 50 or loaded into the scaffold after it is formed. The variety of different therapeutic agents that can be used in conjunction with the scaffold 10, 30, 50 of the present invention is vast. In general, therapeutic agents which may be administered via the scaffold 10, 30, 50 include, without limitation: antiinfectives such as antibiotics and antiviral agents; chemotherapeutic agents (i.e. anticancer agents); anti-rejection agents; analgesics and analgesic combinations; anti-inflammatory agents; hormones such as steroids; growth factors (bone morphogenic proteins (i.e. BMPs 1-7), bone morphogenic-like proteins (i.e. GFD-5, GFD-7 and GFD-8), epidermal growth factor (EGF), fibroblast growth factor (i.e. FGF 1-9), platelet derived growth factor (PDGF), insulin like growth factor (IGF-I and IGF-II), transforming growth factors (i.e. TGF-β I-III), vascular endothelial growth factor (VEGF); and other naturally derived or genetically engineered proteins, polysaccharides, glycoproteins, or lipoproteins. These growth factors are described in The Cellular and Molecular Basis of Bone Formation and Repair by Vicki Rosen and R. Scott Thies, published by R.G. Landes Company.

Composite scaffolds 10, 30, 50 containing bioactive materials may be formulated by mixing one or more therapeutic agents with the polymes used to make the construct, with the solvent, or with the polymes-solvent mixture that is then foamed via lyophilization. Alternatively, a therapeutic agent may be coated on the composite scaffold 10, 30, 50 with a pharmaceutically acceptable carrier that does not dissolve the scaffold 10, 30, 50. The therapeutic agents, may be a liquid, a finely divided solid, or any other appropriate physical form. Typically, but optionally, the matrix will include one or more additives, such as diluents, carriers, excipients, stabilizers or the like. The type of polymes, e.g., 14 and drug concentration can be varied to control the release profile and the amount of drug dispensed. Upon contact with body fluids, the drug will be released. If the drug is incorporated into the foam, then the drug is released as the foam undergoes gradual degradation (mainly through hydrolysis). This can result in prolonged delivery (over, say 1 to 5,000 hours, preferably 2 to 800 hours) of effective amounts (say, 0.0001 mg/kg/hour to 10 mg/kg/hour) of the drug.

As outlined in Vacanti, U.S. Patent No. 5,770,417, cells can be harvested from a patient (before or during surgery to repair the tissue) and the cells can be processed under sterile conditions to provide a specific cell type (i.e., pluripotent cells, stem cells, marrow cells, progenitor human autologous adipose tissue (PHAAT) cells or precursor cells, such as, the mesenchymal stem cells described in Caplan, U.S. Patent No. 5,486,359). These cells, e.g., myocytes, adipocytes, fibromyoblasts, ectodermal cell, muscle cells, osteoblast (i.e. bone cells), chondrocyte (i.e. cartilage cells), endothelial cells, fibroblast, pancreatic cells, hepatocyte, bile duct cells, bone marrow cells, neural cells, genitourinary cells (including nephritic cells) and combinations thereof may be applied or seeded into the porous composite scaffold 10, 30, 50. Autogenous, allogeneic, xenogeneic cells may be used. The cells may be cultured ex vivo and then reimplanted. Tissue may be harvested from a patient, processed to select certain cells and/or growth factors, such as PRP (platelet rich plasma), and then reimplanted with the scaffolds 10, 30, 50 back into the patient. The implanted cells could also contain inserted DNA encoding a protein that could stimulate the attachment, proliferation or differentiation of tissue.

Cells may be implanted into the scaffold 10, 30, 50 by placing the scaffold in a cell culture such that the cells invade the micropores and macropores. The scaffold can then be implanted into the patient. The *in vitro* seeding of cells could provide for a more rapid development and differentiation process for the tissue. It is clear that cellular differentiation and the creation of tissue specific extracellular matrix is critical for the tissue engineering of a functional implant. It is known that different cell types (stromal cells and chondrocytes) can be cultured on different structures. A gradient structure also allows for co-cultured tissue scaffolds 10, 30, 50 to be generated.

One use of the construct described herein is for the repair and regeneration of articular cartilage. Articular cartilage is an example of a naturally occurring structure composed of four different zones that include the superficial or tangential zone within the first 10-20% of the structure (this includes the articular surface), the middle zone, which is 40-60% of the middle structure, the deep zone that is adjacent to the tide mark, and a transition zone between the bone and cartilage that is composed of calcified cartilage. Subchondral bone is located adjacent to the tide mark and this transitions into cancellous bone.

As described above, the present invention permits the fabrication of a scaffold, e.g., 50 having multiple layers. Because the process described above for forming multiple layers can be executed numerous times, the resultant scaffold may have a selected number of layers, each having its own characteristics of composition, porosity, strength, etc. Accordingly, the scaffold, e.g., 50 may act as a template for multiple distinct tissue zones as are present in articular cartilage.

The surface porosity of the foam can be controlled by various methods including providing a mold therefore having a plurality of upstanding pins for piercing the surface during molding or subsequently piercing the surface by needles, laser treatment, chemical treatment, etc., resulting in surface porosity ranging from impervious to porous thereby determining fluid permeability. With regard to fabricating a scaffold, e.g., 10, for repairing articular cartilage, the scaffold 10 may have three zones, viz., a porous polymeric phase 12 which lies adjacent to cartilage tissue, a porous ceramic phase 14 which lies adjacent to bone tissue, and an interphase region 16. The phase 12 would have an upper surface (skin) may be provided with a porosity, e.g., 75 to 150 µm to enable the passage of cells to promote ingrowth. For articular cartilage, the polymer phase 12 and ceramic phase 14 will need to support mechanical loading and thereby protect the invading cells until they have differentiated and consolidated into tissue that is capable of sustaining load. The polymer phase 12 may have a porosity of about 80 to about 95 percent with pores that are of the order of 100 µm (about 80 µm to about 120 µm). It is expected that chondrocytes will invade this zone. The ceramic phase 14 may have larger pores (about 250µm to about 400 µm) and a porosity in the range of about 50 to about 95 percent which is structurally compatible with cancellous bone. The interphase region 16 resembles the structural transition between cartilage and bone.

Several patents have proposed systems for repairing cartilage that could be used with porous scaffolds of the present invention. For example, U.S. Patent No. 5,769,899 describes a device for repairing cartilage defects and U.S. Patent No. 5,713,374, describes securing cartilage repair devices with bone anchors.

The scaffold 10, 30, 50 described herein may also be used for the repair and regeneration of intervertebral discs with the scaffold 10, 30, 50 synthetically re-creating the layered structure of the spinal disc. Reinforcements, e.g., 38, 70 added to the polymeric phase 12, 60 and sandwiched between the two ceramic phases may be used to simulate the complex collagen orientation found in annulus fibrosis of the spinal disc. Such reinforcements provide additional resistance to impact, and the orientation of the reinforcement can be varied to control the mechanical properties of the construct in a manner similar to the way that collagen orientation affects those of the spinal disc.

It should be understood that the embodiments described herein are merely exemplary and that a person skilled in the art may make many variations and modifications without departing from the spirit and scope of the invention as defined in the appended claims. Accordingly, all such variations and modifications are intended to be included within the scope of the invention as defined in the appended claims. For example, the phases, e.g.,12, 14, 32, 34, 36, 52, 60, etc. may have varying thicknesses, as well as varying composition and porosity and may be open or closed cell.

The following examples are illustrative of the principles and practice of this invention, although not limited thereto. Numerous additional embodiments within the scope and spirit of the invention will become apparent to those skilled in the art. In these examples certain abbreviations are used such as PCL to indicate polymerized ε-caprolactone, PGA to indicate polymerized glycolide, PLA to indicate polymerized (L)lactide. Additionally, the percentages in front of the copolymer indicate the respective mole percentages of each constituent.

### Example 1

This example describes the preparation of a bi-layered scaffold composed of a porous polymer phase, e.g. 12, lyophilized in the presence of a porous ceramic phase, e.g. 14.

A solution of the polymer to be lyophilized into a foam was first prepared. In this example, a 95/5 weight ratio of 1,4-dioxane/(35/65 PCL/PGA) was weighed out. Next, the polymer and solvent were placed into a flask, which in turn was put into a water bath and stirred at 70°C for 5 hrs. Afterwards, the solution was filtered using an extraction thimble (extra coarse porosity, type ASTM 170-220 (EC)) and stored in a flask.

The next step was to prepare the porous ceramic phase 14. A porous ceramic tablet (15 mm X 15 mm X 1 mm) was cut from a larger block (#97CAM03/2B, CAM Implants, by Leiden, Netherlands) using a high speed diamond cutting saw (Isomet 1000 series, blade by Beuhler) operating at speeds of 300 - 350 rpm.

A set of 1.0-mm x 6.3-mm x 130-mm shims was placed in a 126-mm X 136-mm aluminum mold with a lip height of 16.4 mm. The shims were placed parallel to each other, 13 - 14.5 mm apart. The ceramic tablet was placed on the shims. The contact between the ceramic tablet and the supporting shims was minimized to overlap less than 1 mm in order to expose the maximum amount of the ceramic tablet to the polymeric solution.

The solution was then added to a level of 1.67 mm in the mold, submerging the bottom face of the ceramic tablet.

The mold assembly was then placed on the shelf of the lyophilizer (or freeze dryer), and the freeze dry sequence begun. A laboratory scale lyophilizer (Model Freeze Mobile G from Virtis Company (Gardiner, NY), was used. The freeze dry sequence used in this example was: 1) 20°C for 15 minutes; 2) -5°C for 120 minutes; 3) -5°C for 90 minutes under vacuum 100 mT; 4) 5°C for 90 minutes under vacuum 100 mT; 5) 20°C for 90 minutes under vacuum 100 mT. As the solution in the mold freeze dried, the size of the polymeric phase shrank, leaving a 1 mm thick polymer phase 12 and an interphase region 16, less than 200 microns, of polymeric foam infiltrating the porous ceramic phase (tablet) 14.

After the cycle was completed, the mold assembly was taken out of the freeze dryer and allowed to degas in a vacuum hood for 2 - 3 hours. The scaffold 10 was then stored under nitrogen.

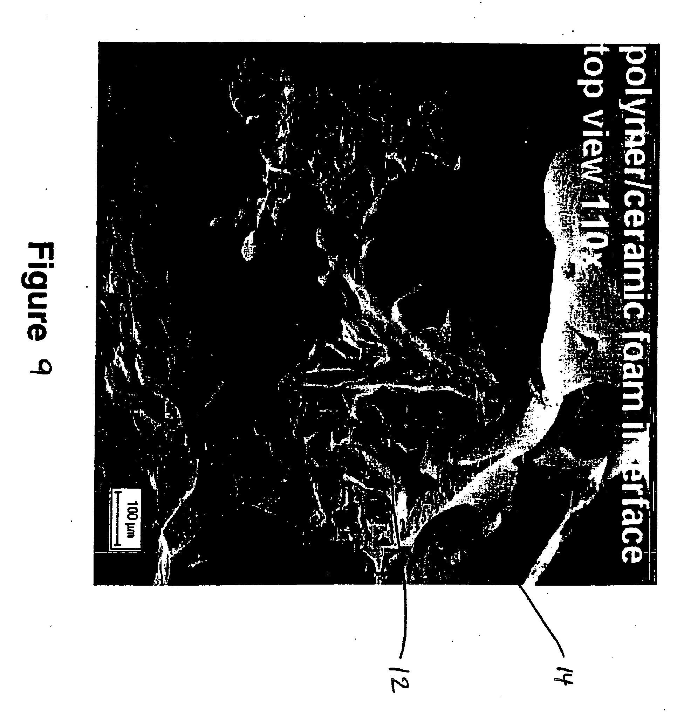

In the resulting scaffold, the ceramic phase 14 was bound firmly to the polymer phase 12. Figure 9 is a scanning electron micrograph (SEM) of the cross-section of the polymer foam embedded in the porous ceramic. The SEM clearly shows both the macroporous ceramic 14 and the microporous polymer 12 foam in the interphase region 16 of the composite scaffold 10.

### Example 2

This example describes the preparation of a bi-layered scaffold such as 30 (See Figures 3 and 4) composed of a porous polymer phase 34 lyophilized in the presence of a porous ceramic phase 32, wherein the structure also contains a biodegradable reinforcing mesh 3 8.

The polymer solution was prepared as described in Example 1. A ceramic tablet, 16.0-mm X 16.9-mm X 8.3-mm, was cut from a larger block as described in Example 1. In addition, an approximately 1-mm deep annular groove was cut into one of the 16.0-mm X 16.9-mm faces of the block using a 10.5-mm circular cutter.

A polydioxanone (PDS) mesh (Ethicon Inc., Somerville, NJ) was cut into strips with approximate dimensions of 11 mm X 50 mm. A strip was loosely cylindrically wound over a 9.5-mm outer diameter rod and secured in a ring configuration by a hot Aaron-ram fine tip (Aaron Medical Industries, Inc., St. Petersburg, FL) that was touched to the mesh at either end of the cylinder to weld the overlapping mesh.

The PDS mesh ring was placed into the groove cut in the ceramic tablet. A set of 5.0-mm x 6.3-mm x 110-mm shims was placed in a 114-mm X 114-mm aluminum mold with a lip height of 16.4 mm. The shims were placed parallel to each other, 13 - 14.5 mm apart. The prepared ceramic piece was placed on the shims with the mesh ring down.

The contact between the ceramic tablet and the supporting shims was minimized with an overlap of 1 mm or less in order to expose the maximum amount of the ceramic tablet to the underlying polymeric solution.

The polymer solution was then added such that the solution reached a level of 5.8 - 6.0 mm in the mold, submerging the bottom face of the ceramic tablet.

The mold assembly was then placed on the shelf of the lyophilizer described in Example 1, and the freeze dry sequence described in Example 1 was followed. After the cycle was completed, the mold assembly was taken out of the freeze dryer and allowed to degas in a vacuum hood for 2 - 3 hours. The scaffold was then stored under nitrogen.

In the resulting scaffold, the ceramic tablet was securely bound to the polymer foam. The mesh was securely fixed in the original position, providing added structural integrity to the scaffold.

### Example 3

This example describes the preparation of a tri-layered scaffold, e.g., like scaffold 50, but without truss 70 (See Figure 5), composed of a porous polymer phase 10 sandwiched between two porous ceramic phases 52, 54.

The polymer solution was prepared as described in Example 1. Two ceramic tablets (~12 mm X ~12 mm X ~2 mm) were cut from a larger block as described in Example 1.

A first ceramic tablet was placed on the bottom of an aluminum mold with a lip height of 18 mm and a diameter of 70 mm. A pair of 2.0-mm x 6.3-mm x 45-mm shims was placed on the bottom of the mold beside the ceramic tablet, parallel thereto and in close proximity. A second set of identical shims was placed over the first set of shims perpendicular thereto, ~10 mm apart and over opposed top edges of the ceramic tablet. The second ceramic tablet was placed on the second set of shims, aligned with the first ceramic tablet forming a 2-mm space between the two ceramic tablets.

The contact between the ceramic tablets and the supporting shims was minimized to overlap less than 1 mm in order to expose the ceramic tablets to the polymeric solution. The polymeric solution was then added to the mold to a level of ~6 mm, submerging the first tablet and contacting the bottom face of the second ceramic tablet.

The mold assembly was then placed on the shelf of the lyophilizer described in Example 1, and the freeze dry sequence begun. The freeze dry sequence used in this example was: 1) 20°C for 15 minutes; 2) -5°C for 120 minutes; 3) -5°C for 120 minutes under vacuum 100 mT; 4) 5°C for 120 minutes under vacuum 100 mT; 5) 20°C for 120 minutes under vacuum 100 mT.

After the cycle, the scaffold was cut out of the polymer using a scalpel. The tri-layer scaffold was placed in an Acetone bath to a depth of 0.5 mm for approximately 1 second to remove the polymer material on the bottom surface of the scaffold to expose the ceramic phase.

### Example 4

A tri-layered scaffold having a lyophilized porous polymer phase sandwiched between two porous ceramic phases, wherein the scaffold also contains a biodegradable reinforcing truss may be fabricated in accordance with the same basic procedure outlined in Example 3. The truss used in this Example has a ring form such as that shown in Figure 3, element 38. The adjacent faces of the ceramic tablets are inscribed with a circular recess as described above in Example 2. A PDS mesh ring of diameter 11mm and 2mm thickness is prepared as in Example 2 and opposing ends thereof are inserted into the circular recesses formed in the adjacent faces of the ceramic layers that are stacked in a mold as in Example 3. The polymer is charged in the mold, lyophilized and cleaned from the bottom surface of the scaffold as in Example 3.

## Claims

1. A composite scaffold, comprising:
a ceramic phase having a first plurality of pores; and
a polymer phase having a second plurality of pores, said polymer phase being attached to said ceramic phase at an interphase region, said polymer phase being infused at least partially into said first plurality of pores in said interphase region.

2. The scaffold of Claim 1, wherein said polymer phase in said interphase region has a portion of said second plurality of pores.

3. The scaffold of Claim 2, wherein said portion of said second plurality of pores communicate at least partially with said first plurality of pores in said interphase region.

4. The scaffold of Claim 3, wherein said pores of said first plurality of pores are larger than said pores of said second plurality of pores.

5. The scaffold of Claim 4, wherein said polymer phase is a polymer foam.

6. The scaffold of Claim 5, wherein said ceramic phase is a first said ceramic phase and said interphase region is a first said interphase region and further including a second said ceramic phase attached to said polymer phase distal to said first ceramic phase through a second said interphase region distal to said first interphase region.

7. The scaffold of Claim 6, further including a mechanical reinforcement structure embedded in said polymer phase.

8. The scaffold of Claim 7, wherein said reinforcement structure is a PDS mesh ring extending generally perpendicularly between said first ceramic phase and said second ceramic phase, said ring inserting partially into a channel formed in adjacent faces of said first and second ceramic phases, thereby extending into said first and second interphase zones.

9. The scaffold of Claim 1, further including a film, scrim, woven textile, non-woven textile, knitted textile, braided textile or truss mechanical reinforcement structure embedded in said polymer phase.

10. The scaffold of Claim 1, further including a growth factor or a therapeutic material as a filler within said polymer phase.

11. The scaffold of Claim 1, further including living cells residing on a surface of said scaffold.

12. The scaffold of Claim 1, wherein at least one of said polymer phase and said ceramic phase is biodegradable.

13. The scaffold of Claim 1, wherein said ceramic is hydroxyapatite, tricalcium phosphate, tetracalcium phosphate, fluoroapatite, magnesium calcium phosphate, calcium sulfate, calcium fluoride, calcium oxide or calcium carbonate.

14. The scaffold of Claim 1, wherein said polymer is a biopolymer collagen, elastin, hyaluronic acid, chitin or alginate.

15. The scaffold of Claim 1, wherein said polymer is an aliphatic polyester homopolymer or an aliphatic polyester copolymer.

16. The scaffold of Claim 15, wherein said polymer is lactic acid, a lactide mixture of L-, D-, meso and D,L lactides, glycolic acid, glycolide, epsilon-caprolactone, p-dioxanone (1,4-dioxan-2-one) or trimethylene carbonate (1,3-dioxan-2-one).

17. The scaffold of Claim 1, wherein said polymer is an aliphatic polyester elastomeric copolymer.

18. The scaffold of Claim 17, wherein said copolymer is formed from epsilon-caprolactone and glycolide in a mole ratio of from 35:65 to 65:35, preferably from 45:55 to 35:65.

19. The scaffold of Claim 17, wherein said copolymer is formed from epsilon-caprolactone and an L-lactide, D-lactide or lactic acid copolymer in a mole ratio of epsilon-caprolactone to lactide of from 35:65 to 65:35, preferably from 45:55 to 30:70.

20. The scaffold of Claim 17, wherein said copolymer is formed from epsilon-caprolactone and an L-lactide, D-lactide or lactic acid copolymer in a mole ratio of epsilon-caprolactone to lactide of from 95:5 to 85:15.

21. A method for making a composite scaffold having a porous ceramic phase and a porous polymer phase, comprising the steps of:
(A) providing a porous ceramic body;
(B) providing a polymer solution;
(C) placing said porous ceramic body in contact with said polymer solution;
(D) permitting said polymer solution to at least partially infuse into pores in said ceramic body;
(E) foaming said polymer solution to produce a polymer foam thereby forming the porous polymer phase, the polymer phase interlocking with the ceramic body where the polymer solution was permitted to infuse into the ceramic body.

22. The method of Claim 21, wherein said step of foaming is by lyophilization.

23. The scaffold of any one of Claims 1 to 20 for repairing a defect area at the gradient junction of cartilaginous tissue and bony tissue, such as articular cartilage, a spinal disc or the meniscus.
